# EUROPEAN PATENT SPECIFICATION

(11) **EP 2 023 821 B1**
(45) Date of publication and mention of the grant of the patent: **07.01.2015**
(21) Application number: 07797619.9
(22) Date of filing: 21.05.2007
(51) Int. Cl.: A61B 10/00, A61M 1/00, B01L 3/00, A61B 1/012, A61B 10/02

(54) **SYSTEM FOR TISSUE SPECIMEN COLLECTION**
SYSTEM ZUR ENTNAHME VON GEWEBEPROBEN
SYSTÈME DE RECUEIL D'ÉCHANTILLONS DE TISSUS

(30) Priority: 19.05.2006 US 438092
(43) Date of publication of application: 18.02.2009
(73) Proprietor: Bracco Diagnostics Inc., Princeton NJ 08540 (US)
(72) Inventor: CUSHNER, Jeffrey, B., Woodmere, NY 11598 (US); KUTZYNA, Peter, M., Exton, PA 19341 (US); WOLCOTT, Kenneth, E., Centerport, NY 11721 (US)
(74) Representative: Ravizza, Claudio
(86) International application number: PCT/US2007/069358
(87) International publication number: WO 2007/137219

(56) References cited:
- EP-A- 0 931 519
- DE-U1- 8 609 830
- GB-A- 2 323 288
- JP-A- 7 079 997
- JP-A- 2000 279 418
- US-A- 3 863 624
- US-A- 4 393 879
- US-A- 4 685 472
- US-A- 5 554 151
- US-A- 5 630 939
- US-A1- 2002 065 474
- US-A1- 2003 069 543
- US-A1- 2003 130 594
- US-B1- 6 361 505
- US-B1- 6 428 316

## Description

### FIELD OF THE INVENTION

The various embodiments of the present invention relate generally to tissue collection devices for retaining a tissue specimen drawn from a patient by a suction device during, for example, an endoscopy procedure.

### BACKGROUND OF THE INVENTION

Endoscopic medical procedures often result in the capture of tissue specimens (such as polyps) recovered from a collection tube in communication with an endoscope during an endoscopy procedure. For example, clinicians often draw tissue specimens through an endoscope and into a collection canister via the application of suction from a suction source positioned proximal to the endoscope. Such tissue specimens are often transported to a pathology laboratory in order to generate a diagnosis based on an analysis of the tissue specimen.

Various conventional tissue specimen traps have been developed for placement between the patient and the collection canister (and a corresponding suction source) in an attempt to separate the tissue specimen from suction effluent that accompanies the tissue specimen from the endoscope. For example, some conventional tissue specimen traps include a "sputum trap" arrangement including a cup or reservoir having a lid defining an inlet (leading to the endoscope) and an outlet (leading to the suction source). Such sputum trap tissue specimen traps may also include one or more baskets or chambers disposed near the inlet for retaining tissue specimens that are drawn into the sputum cup by the suction. However, conventional "sputum cup" tissue specimen traps do not provide direct co-axial fluid communication between the endoscope and the suction source (as both the inlet and outlets of the cup are defined in a "lid" or "cover" of the trap). Thus, suction effluent that accompanies the tissue specimen may not be effectively separated from the tissue specimen retained in the basket or chamber of the trap due to the indirect suction applied to the tissue specimen. For example, the reservoir or "cup" of such conventional traps often retains a considerable volume of potentially contaminating effluent. Furthermore, some conventional sputum cup tissue specimen traps that include multiple tissue specimen chambers (or selectable "baskets"), require the removal of the tissue specimen by an accessory device such as a pair of tweezers.

Some additional conventional tissue specimen traps have been developed to retain a tissue specimen in a position that is substantially co-axial with the suction source and the collection tube. For example, clinicians may insert gauze-like "filters" into a proximal portion of a section of tubing (in communication with the endoscope, for example) using a mandrel or other device. The flexible, porous "filter" is removably engaged between adjacent sections of tubing by the peripheral edges of the "filter" material (which may extend outside the tubing and be retained, for example, by the interaction of serially-engaged sections of endoscope tubing). Such conventional tissue specimen traps, however require a clinician to pull out the tissue specimen out of the gauze-like "filter" retained within the tube or to pull out the entire gauze-like "filter." Such additional operations may compromise the tissue specimen thereby limiting its value as a diagnostic indicator when examined, for example, by a pathologist. For example, removal tissue specimen from the gauze-like filter could result in the tissue specimen being dropped. In addition, any handling the tissue specimen requires the clinician to immediately transfer the tissue specimen to a secondary container containing a preservative fluid (such as formalin). In addition, the use of flexible fabric or gauze to construct the filter may result in the unwanted retention of effluent in the filter material which may accompany the tissue specimen as it is transferred for downstream transport and/or analysis steps.

The various complications and additional steps required to process tissue specimens retained in conventional tissue specimen traps may thus not only compromise the tissue specimen, but may also result in confusion and/or misidentifying an anatomical location from which the tissue specimen was taken. For example, in multi-chambered "sputum cup" tissue specimen traps, the clinician may be required to quickly retrieve a tissue specimen from one or more chambers or baskets included in the trap. Thus, the clinician may not have adequate time to note the anatomical location from which each tissue specimen may have been drawn. Thus, downstream analysis of the tissue specimen (by an off-site pathologist, for example) may be compromised by mislabeling and/or misidentification of tissue specimens retained in conventional tissue specimen traps.

Thus, there exists a need in the art for a system and method for tissue specimen collection that addresses at least some of the technical issues associated with conventional tissue specimen traps. For example, there exists a need for a single in-line tissue specimen trap that allows for the effective removal of effluent from a tissue specimen retained in the trap and allows a clinician to easily remove the single tissue specimen trap from serial engagement between a collection tube (such as a section of tubing in fluid communication with an endoscope) and a suction source tube and replace with a second single tissue specimen trap. There further exists a need for a tissue specimen trap that may be used as a container for preserving and/or segregating the tissue specimen as it is transported to a pathology laboratory or other facility. Furthermore, there exists a need for a system and method that allows clinicians to easily organize a plurality of tissue specimen traps used in one or more endoscopy procedures based, for example, on an anatomical location from which each tissue specimen is drawn.

### BRIEF SUMMARY OF THE INVENTION

The embodiments of the present invention satisfy the needs listed above and provide other technical advantages as listed below. Embodiments of the present invention may include a tissue specimen collection system comprising a collection device adapted to be removably and serially engaged between a suction tube and a collection tube. The collection device comprises a distal end operably engaged with the collection tube, and a proximal end operably engaged with the suction tube. The collection device also defines a bore extending therethrough, in coaxial relation with the suction tube and the collection tube, for allowing fluid communication between the suction tube and the collection tube. The system further comprises a screen disposed within the bore of the collection device. The screen is configured to retain a tissue specimen drawn through the collection device by and towards the suction tube. The screen defines a plurality of apertures for allowing fluid communication between the collection tube and the suction tube such that any fluid accompanying the tissue specimen is separated and drawn through the screen and into the suction tube, while the tissue specimen is retained by the screen within the collection device.

According to the invention as defined by claim 1, the collection device is configured to be separable into a first segment (including the distal end, for example) and a second segment (including the proximal end and the screen, for example). Thus, upon separation of the first and second segments, the retained tissue specimen may be accessible for retrieval from the screen by a user of the system. In other system embodiments, the collection device may be configured to serve as a transport container for the tissue specimen retained therein. For example, in some such embodiments, the system may further comprise: a proximal end cap configured to sealingly engage the proximal end of the collection device in a substantially fluid-tight manner when the collection device is removed from serial engagement between the suction tube and the collection tube; and a distal end cap configured to sealingly engage the distal end of the collection device in a substantially fluid-tight manner when the collection device is removed from serial engagement between the suction tube and the collection tube. Thus, the cooperation of the collection device, distal end cap, and proximal end cap may ensure that the tissue specimen is retained within the collection device when the collection device is removed from serial engagement between the suction tube and the collection tube.

Additional system embodiments may further comprise a preservation fluid reservoir configured to operably engage at least one of the proximal end and the distal end of the collection device when the collection device is removed from serial engagement between the suction tube and the collection tube. The preservation fluid reservoir defines an aperture for receiving at least one of the proximal end and the distal end of the collection device and comprises a penetrable membrane configured to substantially seal the aperture so as to contain a preservation fluid within the preservation fluid reservoir. The membrane is configured to be capable of being penetrated by at least one of the proximal end and the distal end of the collection device such that the preservation fluid is released into the bore defined in the collection device to preserve the tissue specimen retained therein.

Some system embodiments further comprise a manifold device configured to removably and serially engage the collection device between the suction tube and the collection tube. The manifold device may comprise at least one valve device operably engaged between the distal end of the collection device and the suction tube, wherein the valve device is configured to selectively allow fluid communication between the collection tube and the suction tube via the collection device. In some such embodiments, the manifold device may be configured to removably and serially engage a plurality of collection devices in parallel relation between the suction tube and the collection tube. Thus, the valve device may be further configured to selectively allow fluid communication between the collection tube and the suction tube via at least one of the plurality of collection devices. The manifold device may also define a bypass bore extending therethrough in coaxial relation with the suction tube and the collection tube. The bypass bore is configured to allow fluid communication between the suction tube and the collection tube. According to such embodiments, the valve device may be further configured to selectively allow fluid communication between the collection tube and the suction tube via the bypass bore so as to bypass the collection device (or a plurality of collection devices in parallel relation between the suction tube and the collection tube).

According to some system embodiments of the present invention, the collection device may comprise at least one indicia corresponding to an anatomical region from which the tissue specimen is drawn. Thus, such embodiments may allow a user of the system to identify the anatomical region from which the tissue specimen was drawn during a medical procedure. In various embodiments, the at least one indicia may include, but is not limited to: an alphanumeric indicia (affixed to the collection device, for example); a color; a bar code; a radio-frequency identification (RFID) device; and combinations of such indicia. In order to provide improved organization and/or identification of the collection device (and tissue specimens retained therein) after removal of the collection device from serial engagement between the suction tube and the collection tube, some system embodiments may further comprise an organizer device configured to removably and serially engage the collection device between the suction tube and the collection tube. The organizer device also defines a plurality of apertures for receiving the collection device when the collection device (retaining the tissue specimen, for example) is removed from serial engagement between the suction tube and the collection tube. The plurality of apertures include one or more anatomical indicia corresponding thereto for indicating an anatomical region from which the tissue specimen is drawn such that the anatomical region is identifiable based at least in part on the anatomical indicia.

Various other embodiments may also provide methods for collecting a tissue specimen. In one embodiment, the method comprises providing a collection device adapted to be removably and serially engaged between a suction tube and a collection tube, wherein the collection device defines a bore extending therethrough in coaxial relation with the suction tube and the collection tube. As described herein, the bore is configured to allow fluid communication between the suction tube and the collection tube. The provided collection device also includes a screen disposed within the bore, defining a plurality of apertures for allowing fluid communication between the collection tube and the suction tube. The method also comprises: operably engaging a distal end of the collection device with the collection tube; operably engaging a proximal end of the collection device with the suction tube; drawing a tissue specimen through the collection tube by and towards the suction tube; and retaining the tissue specimen on the screen such that such that any fluid (such as effluent) accompanying the tissue specimen is separated and drawn through the screen and into the suction tube.

Other method embodiments further comprise separating the collection device into a first segment including the distal end and a second segment including the proximal end and the screen, retrieving the retained tissue specimen from the screen, and submerging the retrieved tissue specimen in a preservation fluid. Other method embodiments may include steps for utilizing the provided collection device as a tissue specimen storage and/or transport container. For example, the method may further comprise operably engaging a proximal end cap with the proximal end of the collection device in a substantially fluid-tight manner when the collection device is removed from serial engagement between the suction tube and the collection tube. The method may also comprise operably engaging a distal end cap with the distal end of the collection device in a substantially fluid-tight manner when the collection device is removed from serial engagement between the suction tube and the collection tube, and transporting the retained tissue specimen to a laboratory within the collection device (which, as described herein, is substantially closed by the proximal and distal end caps operably engaged therewith). In order to preserve the retained tissue specimen as it is stored and/or transported within the collection device, various method embodiments of the present invention may also comprise filling the collection device with a preservation fluid (such as formalin, for example) so as to preserve the retained tissue specimen within the collection device.

Thus, the various embodiments of the present invention provide many advantages that may include, but are not limited to: allowing a clinician to quickly change tissue specimen collection traps without having to immediately transfer the tissue specimen to a separate preservation fluid container; providing a self-contained, enclosed tissue specimen collection device which may be stored prior to transport to a pathology laboratory such that multiple tissue specimens collected during a single endoscopy procedure may be stored and transferred at one time; providing a self-contained, small and portable tissue specimen collection device that allows for an improved separation of effluent from the retained tissue specimen via the application of a suction force that is substantially co-axial with the collection device; and providing a system and method for organizing and identifying multiple tissue specimen collection devices based at least in part on the anatomical location from which the tissue specimen is drawn.

These advantages, and others that will be evident to those skilled in the art, are provided in the system for tissue specimen collection of the present invention.

### BRIEF DESCRIPTION OF THE SEVERAL VIEWS OF THE DRAWINGS

Having thus described the invention in general terms, reference will now be made to the accompanying drawings, which are not necessarily drawn to scale, and wherein:
FIG. 1 shows several views of a system comprising a collection device operably engaged with a collection tube;
FIG. 2A shows a system comprising a collection device operably engaged with distal and proximal end caps;
FIG. 2B shows a system according to one embodiment of the present invention comprising a collection device and a collection tube wherein the ends of the collection tube are operably engaged with the distal and proximal ends of the collection tube;
FIG. 3 shows a system comprising a collection device operably engaged with a preservation fluid reservoir;
FIG. 4 shows a system comprising a pair of collection devices operably engaged with a manifold device;
FIG. 5 shows a system according to one embodiment of the present invention comprising a collection device operably engaged with an organizer device defining apertures for receiving collection devices having tissue specimens retained therein;
FIG. 6 shows a system according to one embodiment of the present invention comprising a collection device operably engaged with an alternate organizer device defining apertures for receiving collection devices having tissue specimens retained therein;
FIG. 7 shows a system according to one embodiment of the present invention comprising a collection device operably engaged with a manifold device defining a reservoir;
FIG. 8 shows a system according to one embodiment of the present invention comprising a collection device operably engaged with a manifold device defining a reservoir wherein the collection device is oriented such that a gravity force acts to drain a fluid from the collection device;
FIG. 9 shows a system according to one embodiment of the present invention comprising a collection device operably engaged with an alternative manifold device defining a reservoir wherein the collection device is oriented such that a gravity force acts to drain a fluid from the collection device;
FIG. 10 shows a system according to one embodiment of the present invention comprising a collection device operably engaged with a manifold device via a cartridge device that is slidably engaged with a cartridge aperture defined in the manifold device; and
FIG. 11 shows a cartridge device for receiving the collection device that is adapted to be slidably engaged with a cartridge aperture defined in the manifold device, according to one embodiment of the present invention.

### DETAILED DESCRIPTION OF THE INVENTION

The present inventions now will be described more fully hereinafter with reference to the accompanying drawings, in which some, but not all embodiments of the inventions are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout.

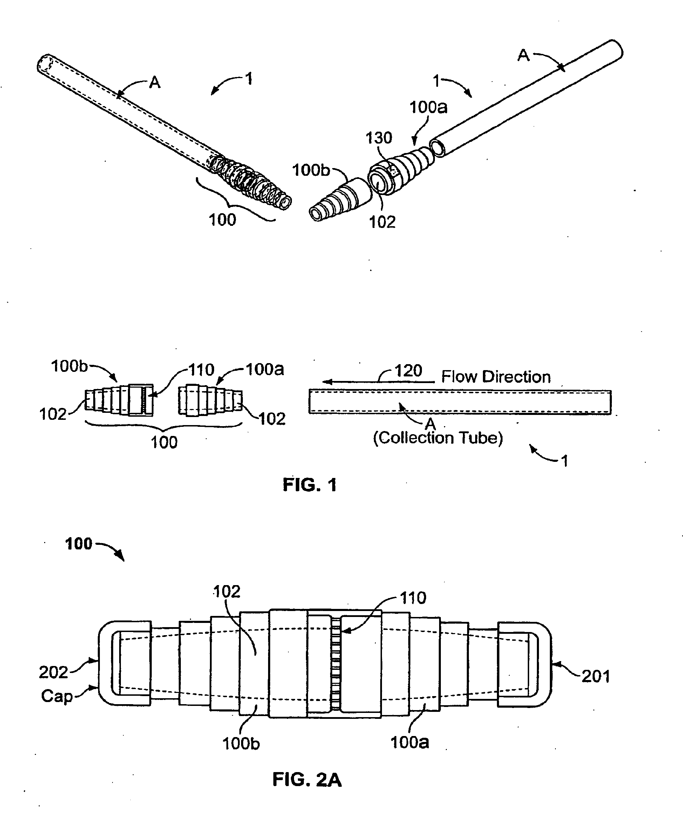

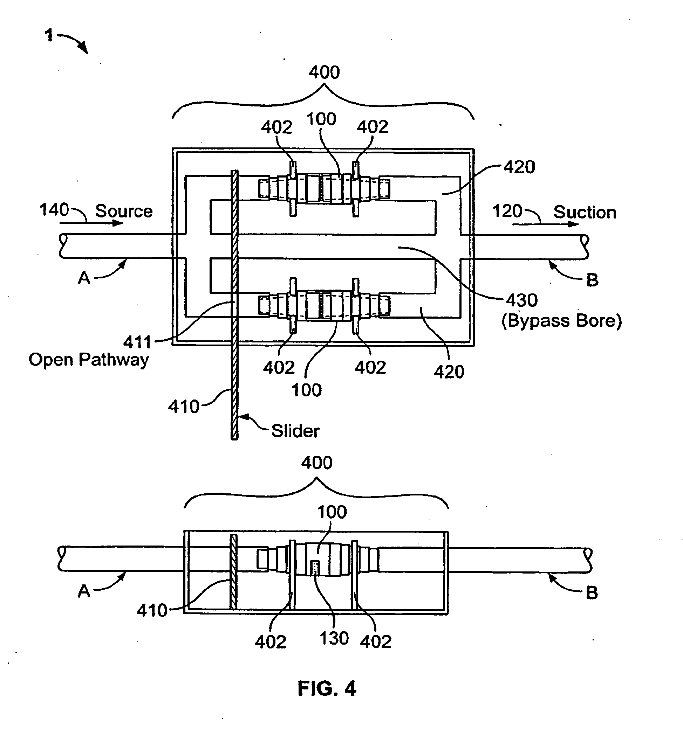

As shown generally in FIG. **1****,** one embodiment provides a tissue specimen collection system **1** comprising a collection device **100** adapted to be removably and serially engaged between a suction tube **B** (see FIG. **4****)** and a collection tube **A.** For example, in some embodiments, the collection device **100** may be adapted to be removably and serially engaged between an endoscope collection tube **A** and a suction tube **B** such that a flow direction **120** is established through the collection device **100** (and/or a bore **102** defined therein) via a suction force imparted by a suction source in fluid communication with the collection device **100** via the suction tube **B** (see FIG. 4).

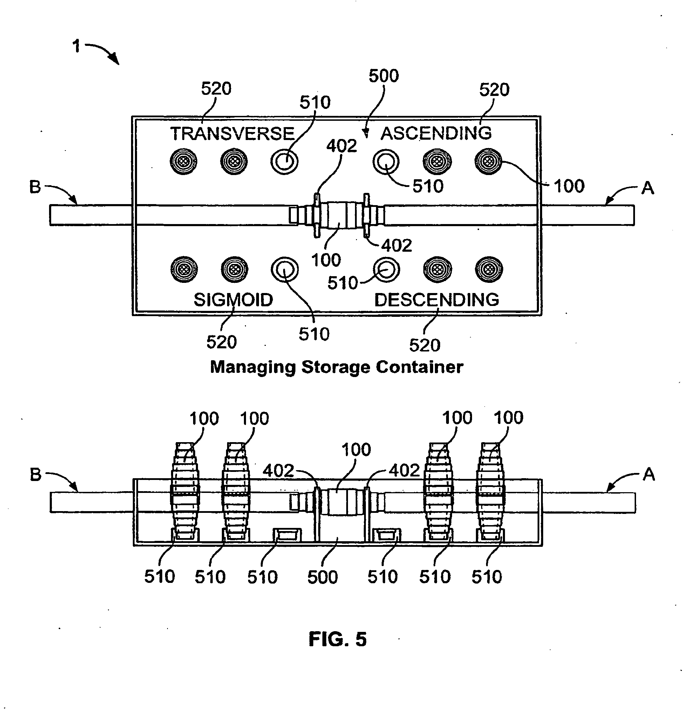

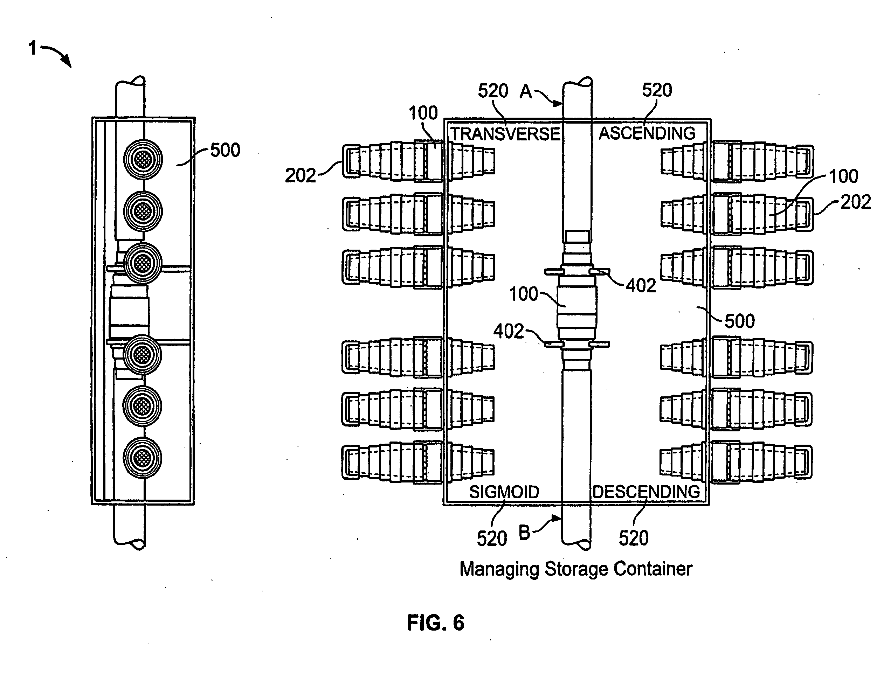

The collection device **100** comprises a distal end operably engaged with the collection tube **A** and a proximal end operably engaged with the suction tube **B** (see FIG. **4****,** for example). As shown generally in FIG. **1****,** the distal and proximal ends of the collection device define one or more steps having varying outer diameters configured to establish an interference fit within collection tubes **A** and/or suction tubes **B** having a variety of inner diameters. In other embodiments, the distal and proximal ends of the collection device may define barbs, threaded surfaces, and/or other quick-connection devices configured to removably and serially engage the collection device **100** between collection tubes **A** and/or suction tubes **B** having a variety of inner diameters. Thus, the collection device **100** may be easily removable from serial engagement between the collection tube **A** and the suction tube **B** by a user of the system 1. When a tissue specimen is retained within the collection device **100** (as described further herein), a user may quickly disengage a collection device **100** retaining a tissue specimen and replace the collection device **100** with an empty collection device **100** that may be relatively easily serially engaged between the collection tube **A** and the suction tube **B.** Thus, according to various system **1** embodiments of the present invention, a single tissue specimen may be captured and retained in a corresponding single collection device **100** that may be organized based upon, for example, an anatomical region from which the tissue specimen is drawn (as discussed further herein with respect to FIGS. **5** and **6** (depicting a system **1** embodiment further comprising an organizer device **500** for organizing filled collection devices **100** after each collection device **100** is removed from serial engagement between the collection tube **A** and the suction tube **B).** The collection device **100** may also be removed from serial engagement between the collection tube **A** and the suction tube **B** and placed directly into a specimen jar or other container containing a preservation fluid (such as formalin, for example) such that the tissue specimen may be contained and submerged for transport.

The collection device **100** defines a bore **102** extending therethrough in coaxial relation with the suction tube **B** and the collection tube **A.** The bore **102** defined by the collection device **100** is configured to allow fluid communication between the suction tube **B** and the collection tube **A.** In some embodiments, the bore **102** may be configured to have an outer dimension (i.e. diameter) substantially equivalent to a diameter of a channel defined in the suction tube **B** and/or the collection tube **A.** In order to retain a tissue specimen, the system **1** further comprises a screen **110** disposed within the bore **102** of the collection device **100.** The screen **110** is configured to retain a tissue specimen drawn through the collection device **100** by and towards the suction tube **B** (i.e. with the flow direction **120** established, for example, by a suction source). The screen **110** defines a plurality of apertures for allowing fluid communication between the collection tube **A** and the suction tube **B** (see FIG. **4****)** such that any fluid accompanying the tissue specimen is separated and drawn through the screen **100** and into the suction tube **B** while the tissue specimen is retained by the screen **110** within the collection device **100.** Because, as shown generally in FIG. **1****,** the collection device **100** defines a bore **102** that is substantially co-axial with flow channels defined by the collection tube **A** and the suction tube **B,** the suction force (establishing a predominant flow direction **120)** may effectively draw effluent and/or other fluids accompanying the tissue specimen through the screen **110** (as substantially all of the cross-sectional area of the screen **110** is exposed to the suction force in the flow direction **120)** while the screen **110** retains the tissue specimen (such as a polyp retrieved during a colonoscopy or other endoscopic procedure). According to various embodiments of the system 1, the apertures defined by the screen **110** may be sized to retain tissue specimens of a selected size while allowing fluids (such as effluents containing small particulates) to pass through the screen **110** and into a collection canister or other waste receptacle downstream of the collection device **100.**

As shown generally in FIG. **1****,** the collection device **100** (and the bore **102** defined therein), may be formed with a substantially circular cross-section. However, in other embodiments, the collection device **100** (and the bore **102** defined therein), may be formed with various cross-sectional shapes that may include, but are not limited to: rectangular, oval, triangular, and combinations thereof. Furthermore, the collection device **100** (and components thereof, such as the screen **110,** for example) may be formed of various materials that may include, but are not limited to: molded polymer; extruded polymer; metal; metallic alloy; and combinations thereof. For example, in one embodiment, the collection device **100** (and the screen **110** disposed therein) may be formed of a substantially transparent, regulatory-compliant polymer (such as medical-grade polycarbonate) such that a clinician may observe the passage of effluent through the bore **102** of the collection device and the retention of a tissue specimen (such as a polyp) by the screen **110** disposed therein. The screen **110** may be integrated with the collection device **100** structure (i.e. integrally formed and/or molded as part of the collection device **100).** In other embodiments, the screen **110** may be formed separately from the collection device **100** and inserted into the bore **102** of the collection device **100** so as to be capable of retaining tissue specimens that are drawn through the collection device **100** (by the suction tube **B,** for example). The screen **110** may thus be formed of the same materials as the structure of the collection device **100.** In other embodiments, the screen **110** may comprise various substantially porous metallic and/or polymer screen materials defining apertures for allowing fluid communication between the sides of the screen **110.**

As described herein, the collection device **100** may also comprise a substantially-transparent tinted polymer such that the polymer is tinted with a color that may be indicative of an anatomical location from which a particular tissue specimen is drawn. Thus, collection devices **100** may be color-coded to match the apertures **510** (corresponding to anatomical indicia **520)** defined in an organizer device **500** (as shown, for example, in FIGS. **5** and **6****).** Thus, the color-coded collection devices **100** may allow for easy identification and placement in the organizer device **500.** Color coding of the collection device **100** may also help eliminate confusion and accidental "mix-ups" of the retained tissue specimen with an incorrect anatomical location. For example, a clinician may serially engage a red-tinted collection device **100** between the collection tube **A** and the suction tube **B** for endoscopy procedures in the sigmoid colon of a patient. Other colors may be used to indicate that a particular collection device **100** contains a tissue specimen drawn from a variety of anatomical locations that may include, but are not limited to: ascending colon; descending colon; transverse colon; and other anatomical regions that may be investigated as part of a colonoscopy or other endoscopy procedure utilizing a collection tube **A** and corresponding suction tube **B.**

Furthermore, the collection device **100** may comprise at least one indicia (such as a marking and/or label operably engaged with a surface of the collection device **100)** corresponding to an anatomical region from which the tissue specimen is drawn such that a user of the tissue specimen collection system **1** may identify the anatomical region from which the tissue specimen was drawn during a medical procedure (such as an endoscopy procedure). According to various embodiments, the at least one indicia of the collection device may include, but is not limited to: an alphanumeric indicia; a color (such as a tint added to a polymer used to form the collection device **100);** a bar code; a radio-frequency identification (RFID) device; and combinations of such indicia. In embodiments wherein the indicia comprises an RFID device integrated with the collection device **100** a user of the system **1** may utilize an RFID transmitter/receiver device (in communication with a computer device, for example) for writing data to the RFID device that may include, but is not limited to: patient identifying information; procedure identifying information (procedure name, time, date, etc.); anatomical region; and/or combinations of such data.

Furthermore, in other embodiments, the collection device **100** may comprise other markings operably engaged therewith. For example, a flow direction indicator (such as an arrow pointing in the intended flow direction **120)** may be printed on a surface of the collection device such that a user of the system **1** may properly serially engage the collection device **100** between the collection tube **A** and the suction tube **B** such that the screen **110** disposed in the bore **102** of the collection device **100** may retain the tissue specimen.

In some system **1** embodiments of the present invention, as shown generally in FIG. **1****,** the collection device **100** is configured to be separable into a first segment **100a** (including the distal end of the collection device **100**), and a second segment **100b** (including the proximal end of the collection device **100** and the screen **110).** Thus, according to such embodiments, the screen **110** may readily accessible to a clinician or technician such that the tissue specimen (retained by the screen **110** disposed within the second segment **100b)** is accessible for retrieval when the first segment **100a** is separated from the second segment **100b.** In some embodiments, the separated second segment **100b** (and the tissue specimen retained on the screen **110** included therein) may also be placed directly into a specimen jar or other container containing a preservation fluid (such as formalin, for example) such that the tissue specimen may be contained and submerged for transport. In other embodiments, once the first segment **100a** is separated from the second segment **100b,** a clinician may retrieve the tissue specimen directly from the screen **110** (using a retrieval tool including, but not limited to: forceps; tweezers; and/or a pipette) and placed directly into a specimen jar or other container containing a preservation fluid such that the tissue specimen may be contained and submerged for transport to a pathology laboratory.

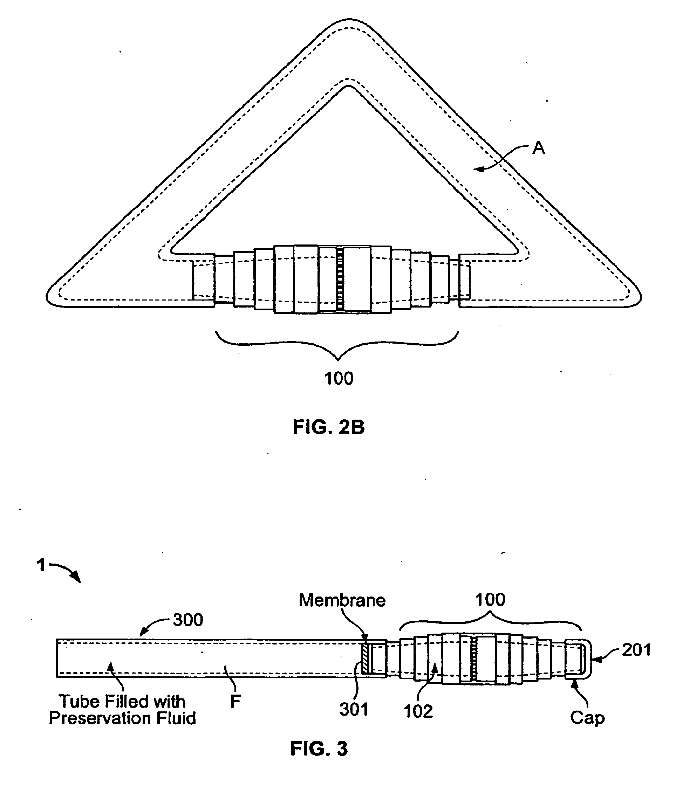

According to some embodiments, as shown generally in FIG. **2A****,** the tissue specimen collection system **1** may further comprise a distal end cap **201** configured to sealingly engage the distal end of the collection device **100** in a substantially fluid-tight manner such that the tissue specimen is retained within the collection device between the screen **110** and the distal end cap **201** when the collection device **100** is removed from serial engagement between the suction tube **B** and the collection tube **A.** Furthermore, as shown in FIG. **2A****,** the system **1** may also further comprise a proximal end cap **202** configured to sealingly engage the proximal end of the collection device **100** in a substantially fluid-tight manner such that a preservation fluid (such as formalin, for example) may be retained within the collection device **100** between the distal end cap **201** and the proximal end cap **202** to preserve the tissue specimen when the collection device **100** is removed from serial engagement between the suction tube **B** and the collection tube **A.** For example, after the collection device **100** is removed from serial engagement between the suction tube **B** and the collection tube **A,** the distal end cap **201** may be operably engaged with the distal end of the collection device **100** to ensure that the tissue specimen retained therein does not emerge from the distal end of the collection device. A preservation fluid (such as formalin, for example) may then be introduced into the bore **102** of the collection device **100** via the open proximal end of the collection device **100** in order to submerge the retained tissue specimen in the preservation fluid. Finally, in order to substantially seal the collection device **100** to serve as a container for transporting and/or storing the retained tissue specimen, the proximal end cap **202** may be operably engaged with the proximal end of the collection device **100** such that the preservation fluid is retained within the collection device **100** between the distal end cap **201** and the proximal end cap **202** during subsequent storage and/or transport. As shown generally in FIG. **2B****,** wherein the collection tube A and/or suction tube **B** comprises a substantially flexible material (such as silicone tubing or other flexible polymer tubing, for example), a length of collection tube A (and/or a length of suction tube **B)** may be cut and bent such that one end of the tube **A, B** is operably engaged with a distal end of the collection device **100** and the other end of the tube **A, B** is operably engaged with the proximal end of the collection device **100.** Thus the tube **A, B** may form a substantially continuous and closed fluid circuit with the bore **102** defined in the collection device **100** such that the retained tissue specimen (and a volume of preservation fluid) may be retained within the collection device **100** (and within the section of tube **A, B).**

FIG. **3** shows an additional system **1** embodiment of the present invention wherein the system **1** further comprises a preservation fluid reservoir **300** configured to operably engage at least one of the proximal end and the distal end of the collection device **100** when the collection device is removed from serial engagement between the suction tube **B** and the collection tube **A.** As shown in FIG. **3****,** the preservation fluid reservoir **300** defines an aperture for receiving at least one of the proximal end and the distal end of the collection device **100.** Furthermore, the preservation fluid reservoir **300** comprises a penetrable membrane **301** configured to substantially seal the aperture so as to contain a preservation fluid **F** (such as formalin, for example) therein. The penetrable membrane **301** is configured to be capable of being penetrated by at least one of the proximal end and the distal end of the collection device **100** such that the preservation fluid **F** is released into the bore **102** defined in the collection device **100** to preserve the tissue specimen retained therein. Thus, as shown in FIG. **3****,** the preservation fluid reservoir **300** may cooperate with a distal end cap **201** to retain the preservation fluid **F** within a channel of the preservation fluid reservoir and/or within the bore **102** of the collection device **100** so as to submerge the retained tissue specimen in the preservation fluid **F** for storage and/or transportation of the collection device **100**/preservation fluid reservoir **300** subassembly.

The aperture defined in the preservation fluid reservoir **300** may be configured to receive and secure at least one end of the collection device **100** in a fluid-tight interference fit. As described herein with respect to the serial engagement of the collection device **100** between the collection and suction tubes **A, B,** the ends of the collection device **100** may comprise a stepped plurality of diameters configured to be capable of being received within a variety of aperture diameters that may be defined by one or more different preservation fluid reservoirs **300.** Furthermore, as shown in **FIG. 3****,** the preservation reservoir **300** may comprise a tube defining a channel having a substantially circular cross-section. The preservation reservoir **300,** however, may comprise a variety of different containers defining an aperture for receiving an end of the collection device **100.** Such container types may include, but are not limited to: vials; bottles; flexible polymer bags; flasks; and combinations of such container types.

FIG. **4** shows an alternate embodiment of the system 1 of the present invention further comprising a manifold device **400** configured to removably and serially engage the collection device **100** between the suction tube **B** and the collection tube **A.** For example, the manifold device **400** may comprise one or more resilient structures **402** (such as elastic C-clips and/or blocks of elastomeric polymer defining apertures for receiving and/or sealing one or more ends of the collection device **100)** for removably engaging the collection device **100** with a body of the manifold device **400.** As shown in FIG. **4****,** the manifold device **400** may comprise at least one valve device **410** operably engaged between the distal end of the collection device **100** and the suction tube **B** and configured to selectively allow fluid communication between the collection tube **A** and the suction tube **B** via the collection device **100** (and/or a bore **102** defined therein). For example, the valve device **410** may comprise a slidable barrier (configured to be slidable in a direction substantially perpendicular to the flow direction **120,** for example) defining a valve aperture **411** sized to substantially approximate the diameter of the bore **102** of the collection device **102.** Thus, when the valve device **410** is positioned to allow fluid communication between the collection tube **A** and the suction tube **B** via the collection device **100** (i.e. by positioning the valve aperture **411** in line with the bore **102** of the collection device **100)** a tissue specimen (and/or fluid accompanying such a specimen) may be drawn through the collection device **100** via the suction force created by the suction tube **100** (and/or a suction source in fluid communication therewith).

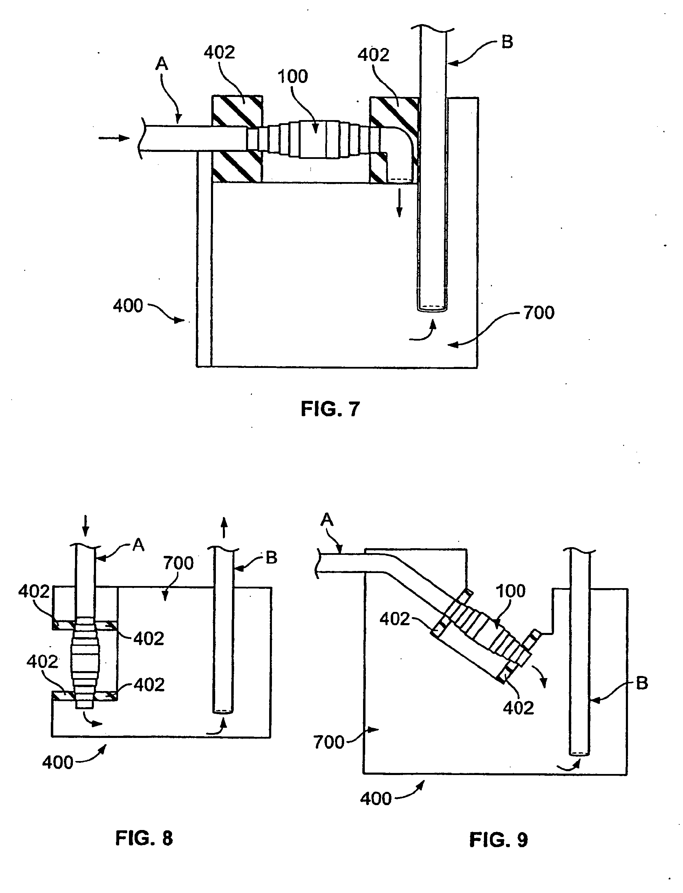

In some embodiments, as shown generally in FIGS. **7-11****,** the manifold device **400** defines a reservoir **700** in fluid communication between the proximal end of the collection device **100** and the suction tube **B.** For example, as shown in FIGS. **7-9****,** the manifold device **400** may define a suction aperture for receiving the suction tube **B** such that the suction tube extends into the reservoir **700.** As described further herein, the proximal end of the collection device **100** may be operably engaged with a substantially resilient structure **402** (such as an elastic polymer material defining an aperture configured to create a fluid tight seal around the proximal end of the collection device **100)** such that substantially leak-free fluid communication may be established between a proximal end of the collection device **100** and an entrance aperture defined in the manifold device **400.** The reservoir **700** may be configured to receive any fluid accompanying the tissue specimen such that the fluid may be drawn into the suction tube **B** by the application of a suction force. Thus, as a tissue specimen is drawn from the collection tube **A** and into the collection device **100** disposed at an entrance to the manifold device **400,** the tissue specimen is retained on the screen **110** within the bore **102** of the collection device **100** and any fluid accompanying the tissue sample is drawn into the reservoir **700** and subsequently through the suction tube **B** by the application of a suction force via the suction tube **B.**

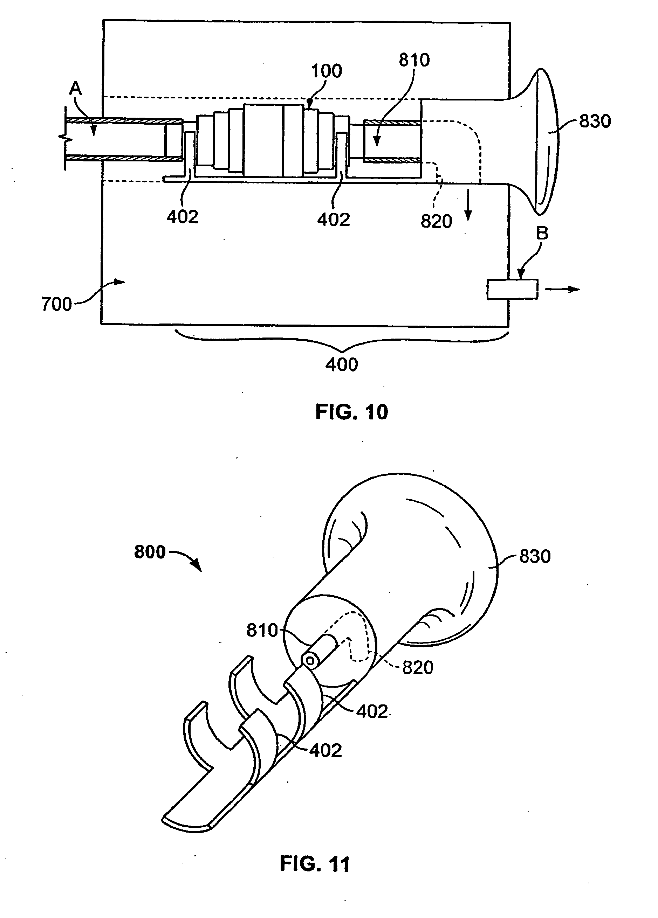

As shown in FIGS. **7-9****,** the manifold device **400** may comprise at least one substantially resilient structure **402** configured to removably and serially engage the collection device **100** between the suction tube **B** and the collection tube **A.** For example, the resilient structure **402** may include, but is not limited to: a substantially-resilient and/or elastic polymer block defining an aperture being configured to receive at least one of the proximal and distal ends of the collection device **100** (such that the polymer block **402** (see FIG. **8****,** for example) may establish fluid communication between the collection tube **A** and the collection device **100;** a substantially-resilient C-clip being configured to receive at least one of the proximal and distal ends of the collection device **100** (see generally, the C-clip resilient structure **402** shown operably engaged with the cartridge device **800** in FIG. **11****);** and combinations of such substantially resilient structures **402.** As shown in FIGS. **8** and **9****,** the manifold device **400** (and the substantially resilient structures **402** included in some embodiments thereof) may be configured to orient the collection device **100** substantially vertically (see generally, FIG. **8****)** and/or at an acute angle (see generally, FIG. **9****)** such that a gravity force may aid the drainage of fluid from the collection device **100** (in addition to the exertion of a suction force via the suction tube **B** for example).

Furthermore, and as shown generally in FIGS. **10** and **11****,** the system **1** may further comprise a cartridge device **800** configured to removably and serially engage the collection device **100** between the reservoir **700** and the collection tube A. The cartridge device **800** may be configured to be slidably disposed in a cartridge chamber defined in a surface of the manifold device **400** such that the cartridge device **800** is selectively movable between a first position (see FIG. **10****,** for example) and a second position relative to the manifold device **400.** For example, as shown in FIG. **10****,** the distal end of the collection **device 100** may be in direct fluid communication with the collection tube **A** (via, for example, an aperture defined in a resilient structure **402** (as shown in FIG. 7)) when the cartridge device is in the first position. Furthermore, the cartridge device **800** may comprise a handle **830** defining an exit channel **810** that may be in fluid communication with a proximal end of the collection device **100.** The exit channel **810,** as shown in FIG. **10****,** may be configured to direct any fluid separated from the retained tissue sample into the reservoir **700** defined by the manifold device **400** via an exit aperture **820** defined in a lower portion of the cartridge device **800.** Furthermore, as shown in FIG. **11****,** the cartridge device **800** may be configured to be slidably removed from the manifold device **400** (and/or slidably extended from the manifold device **400)** such that a user of the system may have access to the substantially resilient structures **402** (such as one or more C-clips) for removing and/or replacing a collection device **100** from the cartridge device **800** during and/or after a medical procedure.

In some system **1** embodiments, the manifold device may be configured to removably and serially engage a plurality of collection devices **100** (such as a pair of collection devices **100,** as shown generally in FIG. **4****)** in parallel relation between the suction tube **B** and.the collection tube **A.** Furthermore, the valve device **410** may be further configured to selectively allow fluid communication between the collection tube **A** and the suction tube **B** via at least one of the plurality of collection devices. Thus, by actuating the valve device **410,** a user (such as a clinician performing and/or assisting in an endoscopy procedure) may select one or more of the collection devices **100** serially engaged (in the manifold device **400)** in parallel relation between the suction tube **B** and the collection tube A. As shown in FIG. **4****,** the manifold device **400** may also define a bypass bore **430** extending therethrough in coaxial relation with the suction tube **B** and the collection tube **A.** The bypass bore **430** is configured to allow fluid communication between the suction tube **B** and the collection tube **A** when, for example, the valve aperture **411** of the valve device **410** is aligned with the bypass bore **430.** As shown in FIG. **4****,** the valve device **410** may be further configured to selectively allow fluid communication between the collection tube **A** and the suction tube **B** via the bypass bore **430** so as to bypass one or more of the plurality of collection devices **100** disposed in parallel within the manifold device **400.**

Thus, according to some system **1** embodiments, as shown generally in FIG. **4****,** manifold device **400** may allow a user to pre-place multiple collection devices **100** in a single manifold device **400.** Furthermore, as described herein, the user may actuate the valve device **410** (such as a manual sliding barrier) to switch between individual collection devices **100** that may be serially engaged (within the manifold device **400)** between the collection and suction tubes **A, B** without having to physically replace individual collection devices **100.** As shown in FIG. **4****,** the manifold device **400** may be generally rectangular and include a slidable valve device **410** defining an aperture **411** that may be actuated linearly so as to be aligned with one or more of the bores **102** defined in the collection devices **100.** In other embodiments, the manifold device **400** may comprise a substantially cylindrical chamber configured to serially engage a plurality of collection devices **100** in parallel relation between the collection and suction tubes **A, B.** According to such embodiments, the valve device **410** may comprise a rotatable disc or other barrier defining a single aperture **411** that may be selectively aligned with one or more of the plurality of collection devices **100** arranged in a substantially circular pattern within the cylindrical manifold device **400.** Furthermore, while the valve devices **410** shown in FIG. **4** is manually operated, it should be understood that the valve device **410** may also comprise one or more pneumatic, electromechanical, and/or solenoid-operated valve devices for selectively allowing fluid communication between the collection and suction tubes **A, B** via one or more individual collection devices **100** disposed within the manifold device **400.**

FIGS. **5** and **6** show an alternative embodiment of the system **1** of the present invention further comprising an organizer device **500** configured to removably and serially engage the collection device **100** between the suction tube **B** and the collection tube **A.** As shown in FIGS. **5** and **6****,** the organizer device **500** may comprise a plurality of resilient structures **402** (such as substantially elastic polymeric and/or metallic "C" clips) for operably engaging the collection device **100** with the organizer device **500** and for serially engaging the collection device **100** between the suction tube **B** and the collection tube **A.** The organizer device **500** defines a plurality of apertures **510** therein for receiving the collection device **100** when the collection device **100** (retaining the tissue specimen) is removed from serial engagement between the suction tube **B** and the collection tube **A.** As shown in FIGS. **5** and **6****,** the plurality of apertures **510** may include one or more anatomical indicia **520** corresponding thereto for indicating an anatomical region from which the tissue specimen is drawn such that the anatomical region is identifiable based at least in part on the anatomical indicia **520.** For example, the embodiment of the organizer device **500** shown generally in FIG. **5** includes twelve apertures **510** divided into four different anatomical regions (each having a corresponding anatomical indicia **520).** Thus, referring to FIG. 5, a collection device **100** containing a tissue specimen drawn from a patient's sigmoid colon may be placed in an aperture **510** in the region of the organizer device **500** corresponding to the "sigmoid" anatomical indicia **520** (i.e. the lower left portion of the organizer device **500** shown in FIG. **5****).** Thus, the organizer device **500** may allow a user to quickly and easily associate retained tissue specimens with the anatomical location from which the specimen was removed. As described herein, the collection devices **100** may be further keyed by color or other indicia **130** to ensure that the collection device **100** is properly placed within the organizer device **500.**

There are also provided methods for collecting a tissue specimen in a collection device disposed between a collection tube **A** (such as a section of tubing in fluid communication with an endoscope or other medical instrument, for example) and a suction source (configured to draw a vacuum and/or create a suction force within a suction tube **B.** According to some embodiments, the method first comprise providing a collection device **100** such as that described herein with respect to the system **1** embodiments of the present invention (shown, for example, in FIGS. **1-6****).** The provided collection device **100** is adapted to be removably and serially engaged between a suction tube **B** and a collection tube **A.** The provided collection device **100** defines a bore **102** extending therethrough in coaxial relation with the suction and collection tubes **B, A.** The bore **102** is configured to allow fluid communication between the suction and collection tubes **B, A.** Furthermore, the provided collection device **100** comprises a screen **110** disposed within the bore **102** defining a plurality of apertures for allowing fluid communication between the collection tube **A** and the suction tube **B.**

The method further comprises operably engaging a distal end of the collection device **100** with the collection tube **A** and operably engaging a proximal end of the collection device **100** with the suction tube **B.** As shown generally in FIG. **1****,** the operably engaging steps may be performed by inserting the ends of the collection device **100** into channels defined in the respective ends of the collection and suction tubes **A, B.** The method further comprises drawing a tissue specimen through the collection tube by and towards the suction tube **B** (for example, by applying a suction force within a channel defined by the suction tube **B** using a suction source (not shown)). The method further comprises retaining the tissue specimen on the screen **110** such that any fluid accompanying the tissue specimen is separated, and drawn through the screen **110** and into the suction tube **B.**

According to some additional method embodiments, the method further comprises separating the collection device **100** into a first segment **100a** including the distal end and a second segment **100b** including the proximal end and the screen **110.** Such method embodiments further comprise: retrieving the retained tissue specimen from the screen **110** (using, for example, a tool such as a pair of tweezers or forceps); and submerging the retrieved tissue specimen in a preservation fluid (such as formalin, for example). The preservation fluid may be contained, for example, in a specimen jar or other container configured to retain the preservation fluid and the retained tissue specimen for storage and/or transport to a pathology laboratory.

Some method embodiments may alternatively comprise utilizing the provided collection device **100** as a container for storing and/or transporting the retained tissue specimen to a pathology laboratory for inspection and/or analysis. For example, such embodiments may comprise operably engaging a distal end cap 201 with the distal end of the collection device **100** in a substantially fluid-tight manner such that the tissue specimen is retained within the collection device **100** (between the screen **110** and the distal end cap **201)** when the collection device **100** is removed from serial engagement between the suction tube **B** and the collection tube **A.** Furthermore, in order to ensure that the collection device **100** is capable of retaining preservation fluid (such as formalin, for example) therein, some method embodiments may further comprise operably engaging a proximal end cap **202** with the proximal end of the collection device **100** in a substantially fluid-tight manner such that the tissue specimen is retained within the collection device **100** between the distal end cap **201** and the proximal end cap **202** when the collection device **100** is removed from serial engagement between the suction tube **B** and the collection tube **A** (see, for example, FIG. **2A****).** The method may also comprise transporting the retained tissue specimen to a laboratory within the collection device **100.** As described herein, various method embodiments of the present invention may also comprise filling the collection device **100** with a preservation fluid prior to operably engaging the proximal end cap **202** with the proximal end of the collection device **100** so as to preserve the retained tissue specimen within the collection device **100** when transporting the retained tissue specimen to the laboratory (such as a pathology laboratory).

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A tissue specimen collection system comprising:
a collection device (100) adapted to be removably and serially engaged between a suction tube (B) and a collection tube (A), the collection device being configured to be separable into a first segment (100a) comprising a distal end operably engaged with the collection tube and and a second segment (100b) comprising a proximal end operably engaged with the suction tube, the collection device defining a bore extending there through in coaxial relation with the suction tube and the collection tube, the bore being configured to allow fluid communication there between;
a screen (110) disposed within the bore of the collection device and positioned in the second segment of the collection device, the screen being configured to retain a tissue specimen drawn through the collection device by and towards the suction tube, the screen defining a plurality of apertures for allowing fluid communication between the collection tube and the suction tube; and
said screen being positioned in the second segment of the collection device such that any fluid accompanying the tissue specimen passing through the collection tube and the first segment of the collection device is separated, and drawn through the screen and into the suction tube, while the tissue specimen is retained by the screen within the bore of the second segment of the collection device, and such that the retained tissue specimen is accessible for retrieval when the first segment is separated from the second segment,
wherein the distal end of the collection device is configured to engage one end of the collection tube or suction tube and the proximal end of the collection device is configured to engage the other end of the collection tube or suction tube, such that the collection tube or suction tube may form a substantially continuous and closed fluid circuit with the bore defined in the collection device.

2. The tissue specimen collection system according to Claim 1, wherein the collection device proximal end comprises barbed fitting for removably attaching the collection device to the suction tube.

3. The tissue specimen collection system according to Claim 1, wherein the collection device proximal end comprises threaded surfaces to removably engage the collection device with the suction tube, said threaded surface configured to accommodate suction tubes of varying inner diameters.

4. The tissue collection system according to Claim 1, wherein the screen is integrally formed as part of the second segment of the collection device.

5. The tissue collection system according to Claim 1, wherein the screen is molded as part of the collection device.

6. The tissue collection system according to Claim 1, wherein the second segment, when removed from the first segment, is configured to enable placement of the second segment into a specimen jar.

7. The tissue collection system according to Claim 1, wherein the collection tube is configured to be removably engaged with an endoscope.

8. The tissue specimen collection system according to Claim 1, wherein the screen is substantially planar.

9. The tissue specimen collection system according to Claim 1, further comprising a manifold device configured to removably and serially engage the collection device between the suction tube and the collection tube.

10. The tissue specimen device according to Claim 9, wherein the manifold device comprises at least one valve device operably engaged between the distal end of the collection device and the suction tube and configured to selectively allow fluid communication between the collection tube and the suction tube via the collection device.

11. The tissue specimen device according to Claim 9, wherein the manifold device defines a reservoir in fluid communication between the proximal end of the collection device and the suction tube, the reservoir being configured to receive any fluid accompanying the tissue specimen such that the fluid may be drawn into the suction tube.

12. The tissue specimen device according to Claim 9, wherein the manifold device comprises at least one substantially resilient structure configured to removably and serially engage the collection device between the suction tube and the collection tube.

13. The tissue specimen device according to Claim 12, wherein the at least one substantially resilient structure is selected from the group consisting of:
a substantially-resilient polymer block defining an aperture being configured to receive at least one of the proximal and distal ends of the collection device;
a substantially-resilient C-clip being configured to receive at least one of the proximal and distal ends of the collection device; and
combinations thereof.

14. The tissue specimen device according to Claim 11, further comprising a cartridge device configured to removably and serially engage the collection device between the reservoir and the collection tube, the cartridge device being slidably disposed in a cartridge chamber defined in a surface of the manifold device such that the cartridge device is selectively movable between a first position and a second position relative to the manifold device;
wherein the distal end of the collection device is in direct fluid communication with the collection tube when the cartridge device is in the first position; and
wherein the collection device is accessible for removal from the cartridge device when the cartridge device is in the second position.

15. The tissue specimen collection system according to Claim 10,
wherein the manifold device is configured to removably and serially engage a plurality of collection devices in parallel relation between the suction tube and the collection tube and wherein the at least one valve device is further configured to selectively allow fluid communication between the collection tube and the suction tube via at least one of the plurality of collection devices.

16. The tissue specimen collection system according to Claim 10,
wherein the manifold device defines a bypass bore extending therethrough in coaxial relation with the suction tube and the collection tube, the bypass bore being configured to allow fluid communication therebetween, and wherein the at least one valve device is further configured to selectively allow fluid communication between the collection tube and the suction tube via the bypass bore so as to bypass the collection device.

17. The tissue specimen collection system according to Claim 1, wherein the collection device comprises at least one indicia corresponding to an anatomical region from which the tissue specimen is drawn such that a user of the tissue specimen collection system may identify the anatomical region from which the tissue specimen was drawn during a medical procedure.

18. The tissue specimen collection system according to Claim 17,
wherein the at least one indicia is selected from the group consisting of:
an alphanumeric indicia;
a color;
a bar code;
a radio-frequency identification (RFID) device; and
combinations thereof.

19. The tissue specimen collection system according to Claim 1, further comprising an organizer device configured to removably and serially engage the collection device between the suction tube and the collection tube, the organizer device defining a plurality of apertures therein for receiving the collection device when the collection device retaining the tissue specimen is removed from serial engagement between the suction tube and the collection tube, the plurality of apertures including one or more anatomical indicia corresponding thereto for indicating an anatomical region from which the tissue specimen is drawn such that the anatomical region is identifiable based at least in part on the anatomical indicia.

## Patentansprüche

1. System zur Entnahme von Gewebeproben umfassend:
eine Sammelvorrichtung (100), die lösbar und seriell zwischen einem Saugrohr (B) und einem Sammelrohr (A) eingebunden werden kann, wobei die Sammelvorrichtung so konfiguriert ist, dass sie in ein erstes Teilstück (100a), das ein mit dem Sammelrohr operabel verbundenes distales Ende aufweist, und ein zweites Teilstück (100b) getrennt werden kann, das ein mit dem Saugrohr operabel verbundenes proximales Ende aufweist, wobei die Sammelvorrichtung eine sich durch sie hindurch erstreckende, in Relation zu dem Saugrohr und dem Sammelrohr koaxiale Bohrung definiert, wobei die Bohrung so konfiguriert ist, dass sie die Übertragung von Flüssigkeiten dazwischen ermöglicht;
einen Filter (110), der innerhalb der Bohrung der Sammelvorrichtung angeordnet ist und der im zweiten Teilstück der Sammelvorrichtung positioniert ist, wobei der Filter zum Zurückhalten einer Gewebeprobe konfiguriert ist, die von und zu dem Saugrohr hin durch die Sammelvorrichtung hindurch gesaugt wird, wobei der Filter eine Vielzahl von Öffnungen definiert, die eine Übertragung von Flüssigkeit zwischen dem Sammelrohr und dem Saugrohr ermöglichen; und
wobei der Filter im zweiten Teilstück der Sammelvorrichtung so positioniert ist, dass sämtliche Flüssigkeit, welche die Gewebeprobe begleitet, die das Sammelrohr und das erste Teilstück der Sammelvorrichtung passiert, abgetrennt wird und durch den Filter hindurch und in das Saugrohr gesaugt wird, während die Gewebeprobe von dem Filter in der Bohrung des zweiten Teilstück der Sammelvorrichtung zurückgehalten wird, und so, dass die zurückgehaltene Gewebeprobe zur Entnahme zugänglich ist, wenn das erste Teilstück vom zweiten Teilstück abgetrennt wird,
wobei das distale Ende der Sammelvorrichtung so konfiguriert ist, dass es mit einem Ende des Sammelrohrs oder des Saugrohrs verbunden werden kann, und das proximale Ende der Sammelvorrichtung so konfiguriert ist, dass es mit dem anderen Ende des Sammelrohrs oder des Saugrohrs verbunden werden kann, so dass das Sammelrohr oder das Saugrohr einen im wesentlichen kontinuierlichen und geschlossenen Flüssigkeitskreislauf mit der Bohrung bilden kann, die in der Sammelvorrichtung definiert ist.

2. System zur Entnahme von Gewebeproben nach Anspruch 1, wobei das proximale Ende der Sammelvorrichtung eine Tülle zum lösbaren Anbringen der Sammelvorrichtung an dem Saugrohr aufweist.

3. System zur Entnahme von Gewebeproben nach Anspruch 1, wobei das proximale Ende der Sammelvorrichtung Gewindeflächen zum lösbaren Verbindung der Sammelvorrichtung mit dem Saugrohr aufweist, wobei die Gewindefläche so konfiguriert ist, dass sie zu Saugrohren mit variierenden inneren Durchmessern passt.

4. System zur Entnahme von Gewebeproben nach Anspruch 1, wobei der Filter integral als Teil des zweiten Teilstücks der Sammelvorrichtung geformt ist.

5. System zur Entnahme von Gewebeproben nach Anspruch 1, bei dem der Filter als Teil der Sammelvorrichtung geformt ist.

6. System zur Entnahme von Gewebeproben nach Anspruch 1, bei dem das zweite Teilstück so konfiguriert ist, dass es in einem Probengefäß untergebracht werden kann, wenn es von dem ersten Teilstück getrennt ist.

7. System zur Entnahme von Gewebeproben nach Anspruch 1, bei dem das Sammelrohr so konfiguriert ist, dass es lösbar mit einem Endoskop verbunden werden kann.

8. System zur Entnahme von Gewebeproben nach Anspruch 1, bei dem der Filter im Wesentlichen planar ist.

9. System zur Entnahme von Gewebeproben nach Anspruch 1, das weiterhin eine Verteilervorrichtung umfasst, die so konfiguriert ist, dass sie die Sammelvorrichtung lösbar und seriell zwischen dem Saugrohr und dem Sammelrohr einbindet.

10. System zur Entnahme von Gewebeproben nach Anspruch 9, bei dem die Verteilervorrichtung mindestens ein Ventilelement enthält, dass operabel zwischen dem distalen Ende des Sammelrohrs und dem Saugrohr eingebunden ist und dass so konfiguriert ist, dass es eine selektive Übertragung von Flüssigkeit zwischen dem Sammelrohr und dem Saugrohr über die Sammelvorrichtung ermöglicht.

11. System zur Entnahme von Gewebeproben nach Anspruch 9, bei dem die Verteilervorrichtung ein Reservoir zur Flüssigkeitsübertragung zwischen dem proximalen Ende der Sammelvorrichtung und dem Saugrohr definiert, wobei das Reservoir so konfiguriert ist, dass es jede Flüssigkeit aufnimmt, welche die Gewebeprobe begleitet, so dass die Flüssigkeit in das Saugrohr gesaugt werden kann.

12. System zur Entnahme von Gewebeproben nach Anspruch 9, bei dem die Verteilervorrichtung mindestens eine im Wesentlichen elastische Konstruktion enthält, die so konfiguriert ist, dass sie die Sammelvorrichtung lösbar und seriell zwischen dem Saugrohr und dem Sammelrohr einbindet.

13. System zur Entnahme von Gewebeproben nach Anspruch 12, bei dem die mindestens eine, im Wesentlichen elastische Konstruktion aus der Gruppe ausgewählt ist, die aus
einem im Wesentlichen elastischen Polymerblock, der eine Öffnung definiert, die so konfiguriert ist, dass sie das proximale, das distale oder beide Enden der Sammelvorrichtung aufnehmen kann;
einem im Wesentlichen elastischen C-Clip, der so konfiguriert ist, dass er das proximale, das distale oder beide Enden der Sammelvorrichtung aufnehmen kann, und
Kombinationen davon besteht.

14. System zur Entnahme von Gewebeproben nach Anspruch 11, das weiterhin ein Einsatzelement enthält, das so konfiguriert ist, dass es das Sammelrohr lösbar und seriell zwischen dem Reservoir und dem Sammelrohr einbindet, wobei das Einsatzelement verschiebbar in einer Einsatzelementkammer angeordnet ist, die in einer Fläche der Verteilervorrichtung so definiert ist, dass das Einsatzelement selektiv zwischen einer ersten Position und einer zweiten Position relativ zu der Verteilervorrichtung bewegbar ist,
wobei das distale Ende der Sammelvorrichtung die direkte Übertragung von Flüssigkeit zu dem Sammelrohr ermöglicht, wenn sich das Einsatzelement in der ersten Position befindet; und
wobei die Sammelvorrichtung zur Entnahme aus dem Einsatzelement zugänglich ist, wenn sich das Einsatzelement in der zweiten Position befindet.

15. System zur Entnahme von Gewebeproben nach Anspruch 10, bei dem die Verteilervorrichtung so konfiguriert ist, dass sie eine Vielzahl von Sammelvorrichtungen lösbar und seriell in paralleler Anordnung zwischen dem Saugrohr und dem Sammelrohr einbindet, und wobei das mindestens eine Ventilelement weiterhin so konfiguriert ist, dass es selektiv eine Flüssigkeitsübertragung zwischen dem Sammelrohr und dem Saugrohr über mindestens eine der Vielzahl von Sammelvorrichtungen erlaubt.

16. System zur Entnahme von Gewebeproben nach Anspruch 10, bei dem die Verteilervorrichtung eine Bypassbohrung definiert, die sich in coaxialer Anordnung zu dem Saugrohr und dem Sammelrohr durch sie hindurch erstreckt, wobei die Bypassbohrung so konfiguriert ist, dass sie eine Flüssigkeitsübertragung dazwischen erlaubt, und wobei das mindestens eine Ventilelement weiterhin so konfiguriert ist, dass es selektiv eine Flüssigkeitsübertragung zwischen dem Sammelrohr und dem Saugrohr über die Bypassbohrung erlaubt, um die Sammelvorrichtung zu umgehen.

17. System zur Entnahme von Gewebeproben nach Anspruch 1, bei dem die Sammelvorrichtung mindestens einen Index enthält, der einem anatomischen Bereich entspricht, aus dem die Gewebeprobe entnommen wurde, so dass ein Anwender des Systems zur Entnahme von Gewebeproben den anatomischen Bereich identifizieren kann, aus dem die Gewebeprobe bei einem medizinischen Verfahren entnommen wurde.

18. System zur Entnahme von Gewebeproben nach Anspruch 17, bei dem der mindestens eine Index aus der Gruppe ausgewählt ist, die aus einem alphanummerischen Index, einer Farbe, einem Strichcode, einem Radiofrequenzidentifizierungs (RFID)-Element und Kombinationen davon besteht.

19. System zur Entnahme von Gewebeproben nach Anspruch 1, das weiterhin eine Organisationsvorrichtung enthält, die so konfiguriert ist, dass sie die Sammelvorrichtung lösbar und seriell zwischen dem Saugrohr und dem Sammelrohr einbindet, wobei die Organisationsvorrichtung darin eine Vielzahl von Öffnungen zur Aufnahme der Sammelvorrichtung definiert, nachdem die die Gewebeprobe enthaltene Sammelvorrichtung aus der seriellen Anordnung zwischen dem Saugrohr und dem Sammelrohr entfernt wurde, wobei die Vielzahl von Öffnungen einen oder mehrere dazu korrespondierende anatomische Indices aufweist, um einen anatomischen Bereich aus dem die Gewebeprobe entnommen wurde zu kennzeichnen, so dass der anatomische Bereich zumindest teilweise anhand der anatomischen Indices identifizierbar ist.

## Revendications

1. Système de recueil d'échantillon de tissu comprenant :
un dispositif de recueil (100) adapté pour être engagé de façon amovible et en série entre un tube d'aspiration (B) et un tube de recueil (A), le dispositif de recueil étant conçu pour pouvoir être séparé en un premier segment (100a) comprenant une extrémité distale coopérant fonctionnellement avec le tube de recueil et en un second segment (100b) comprenant une extrémité proximale coopérant fonctionnellement avec le tube d'aspiration, le dispositif de recueil définissant un alésage qui le traverse en relation coaxiale avec le tube d'aspiration et le tube de recueil, l'alésage étant conçu pour permettre une communication de fluide entre eux ;
un tamis (110) disposé à l'intérieur de l'alésage du dispositif de recueil et placé dans le second segment du dispositif de recueil, le tamis étant conçu pour retenir un échantillon de tissu aspiré à travers le dispositif de recueil par et en direction du tube à)d'aspiration, le tamis définissant une pluralité d'ouvertures pour permettre une communication de fluide entre le tube de recueil et le tube d'aspiration ; et
ledit tamis étant placé dans le second segment du dispositif de recueil de telle sorte que tout fluide accompagnant l'échantillon de tissu passant à travers le tube de recueil et le premier segment du dispositif de recueil est séparé, et aspiré à travers le tamis et jusque dans le tube d'aspiration, alors que l'échantillon de tissu est retenu par le tamis à l'intérieur de l'alésage du second segment du dispositif de recueil, et de telle sorte que l'échantillon de tissu retenu est accessible pour être récupéré lorsque le premier segment est séparé du second segment,
dans lequel l'extrémité distale du dispositif de recueil est conçue pour venir en prise avec une extrémité du tube de recueil ou du tube d'aspiration et l'extrémité proximale du dispositif de recueil est conçue pour venir en prise avec l'autre extrémité du tube de recueil ou du tube d'aspiration, de sorte que le tube de recueil ou le tube d'aspiration peut former un circuit de fluide fermé et sensiblement continu avec l'alésage défini dans le dispositif de recueil.

2. Système de recueil d'échantillon de tissu selon la revendication 1, dans lequel l'extrémité proximale du dispositif de recueil comprend un raccord cannelé pour fixer de façon amovible le dispositif de recueil au tube d'aspiration.

3. Système de recueil d'échantillon de tissu selon la revendication 1, dans lequel l'extrémité proximale du dispositif de recueil comprend des surfaces filetées pour mettre en prise de façon amovible le dispositif de recueil avec le tube d'aspiration, ladite surface filetée étant conçue pour recevoir des tubes d'aspiration ayant des diamètres intérieurs différents.

4. Système de recueil d'échantillon de tissu selon la revendication 1, dans lequel le tamis est formé d'une seule pièce avec le second segment du dispositif de recueil.

5. Système de recueil d'échantillon de tissu selon la revendication 1, dans lequel le tamis est moulé d'une seule pièce avec le dispositif de recueil.

6. Système de recueil d'échantillon de tissu selon la revendication 1, dans lequel le second segment, une fois retiré du premier segment, est conçu pour permettre la mise en place du second segment dans un récipient à échantillon.

7. Système de recueil d'échantillon de tissu selon la revendication 1, dans lequel le tube de recueil est conçu pour venir en prise de façon amovible avec un endoscope.

8. Système de recueil d'échantillon de tissu selon la revendication 1, dans lequel le tamis est essentiellement plan.

9. Système de recueil d'échantillon de tissu selon la revendication 1, comprenant en outre un dispositif collecteur-distributeur conçu pour engager de façon amovible et en série le dispositif de recueil entre le tube d'aspiration et le tube de recueil.

10. Système de recueil d'échantillon de tissu selon la revendication 9, dans lequel le dispositif collecteur-distributeur comprend au moins un dispositif à clapet engagé fonctionnellement entre l'extrémité distale du dispositif de recueil et le tube d'aspiration et conçu pour permettre sélectivement une communication de fluide entre le tube de recueil et le tube d'aspiration via le dispositif de recueil.

11. Système de recueil d'échantillon de tissu selon la revendication 9, dans lequel le dispositif collecteur-distributeur définit un réservoir en communication de fluide entre l'extrémité proximale du dispositif de recueil et le tube d'aspiration, le réservoir étant conçu pour recevoir tout fluide accompagnant l'échantillon de tissu de telle sorte que le fluide peut être aspiré dans le tube d'aspiration.

12. Système de recueil d'échantillon de tissu selon la revendication 9, dans lequel le dispositif collecteur-distributeur comprend au moins une structure essentiellement élastique conçue pour engager de façon amovible et en série le dispositif de recueil entre le tube d'aspiration et le tube de recueil.

13. Système de recueil d'échantillon de tissu selon la revendication 12, dans lequel la au moins une structure essentiellement élastique est choisie parmi le groupe comprenant :
un bloc polymère essentiellement élastique définissant une ouverture qui est conçue pour recevoir au moins l'une des extrémités proximale et distale du dispositif de recueil ;
une attache en C essentiellement élastique étant conçue pour recevoir au moins l'une des extrémités proximale et distale du dispositif de recueil ; et
des combinaisons de ceux-ci.

14. Système de recueil d'échantillon de tissu selon la revendication 11, comprenant en outre un dispositif formant cartouche conçu pour engager de façon amovible et en série le dispositif de recueil entre le réservoir et le tube de recueil, le dispositif formant cartouche étant disposé de façon coulissante dans une chambre à cartouche définie dans une surface du dispositif collecteur-distributeur de telle sorte que le dispositif formant cartouche est mobile sélectivement entre une première position et une seconde position par rapport au dispositif collecteur-distributeur ;
dans lequel l'extrémité distale du dispositif de collecte est en communication de fluide directe avec le tube de recueil lorsque le dispositif formant cartouche est dans la première position ; et
dans lequel le dispositif de recueil est accessible pour être retiré du dispositif formant cartouche lorsque le dispositif formant cartouche est dans la seconde position.

15. Système de recueil d'échantillon de tissu selon la revendication 10, dans lequel le dispositif collecteur-distributeur est conçu pour engager de façon amovible et en série une pluralité de dispositifs de recueil en relation parallèle entre le tube d'aspiration et le tube de collecte et dans lequel le au moins un dispositif à clapet est en outre conçu pour permettre sélectivement une communication de fluide entre le tube de recueil et le tube d'aspiration via au moins un dispositif de recueil de la pluralité de dispositifs de recueil.

16. Système de recueil d'échantillon de tissu selon la revendication 10, dans lequel le dispositif collecteur-distributeur définit un alésage de dérivation s'étendant au travers en relation coaxiale avec le tube d'aspiration et le tube de recueil, l'alésage de dérivation étant conçu pour permettre une communication de fluide entre eux, et dans lequel le au moins un dispositif à clapet est en outre conçu pour permettre sélectivement une communication de fluide entre le tube d'aspiration et le tube de recueil via l'alésage de dérivation de manière à contourner le dispositif de recueil.

17. Système de recueil d'échantillon de tissu selon la revendication 1, dans lequel le dispositif de recueil comprend au moins un indicateur correspondant à une région anatomique d'où l'échantillon de tissu a été prélevé de telle sorte qu'un utilisateur du système de recueil d'échantillon de tissu peut identifier la région anatomique d'où l'échantillon de tissu a été prélevé au cours d'un geste médical.

18. Système de recueil d'échantillon de tissu selon la revendication 17, dans lequel le au moins un indicateur est choisi parmi le groupe comprenant :
un signe alphanumérique
une couleur ;
un code à barres ;
un dispositif d'identification par radiofréquence (RFID) ; et
des combinaisons de ceux-ci.

19. Système de recueil d'échantillon de tissu selon la revendication 1, comprenant en outre un dispositif organiseur conçu pour engager de façon amovible et en série le dispositif de recueil entre le tube d'aspiration et le tube de recueil, le dispositif organiseur définissant une pluralité d'ouvertures pour recevoir le dispositif de recueil lorsque le dispositif de recueil retenant l'échantillon de tissu est retiré de l'engagement en série entre le tube d'aspiration et le tube de recueil, la pluralité d'ouvertures comprenant un ou plusieurs repères anatomiques correspondants pour indiquer une région anatomique d'où l'échantillon de tissu a été prélevé de sorte que la région anatomique peut être identifiée en s'appuyant au moins en partie sur le repère anatomique.
